# EUROPEAN PATENT APPLICATION

(11) **EP 4 555 977 A1**
(43) Date of publication of application: **21.05.2025**
(21) Application number: 24211781.0
(22) Date of filing: 08.11.2024
(51) Int. Cl.: A61F 2/38

(54) **MODULAR FEMORAL KNEE IMPLANT**

(30) Priority: 14.11.2023 US 202363598585 P
(71) Applicant: Howmedica Osteonics Corp., Mahwah, NJ 07430 (US)
(72) Inventor: Connors-Ehlert, Ronald, Houston, 77020 (US); Collazo, Carlos, Old Greenwich, 06870 (US); Syed, Abdul-Nafea, Newark, 07106 (US)
(74) Representative: Keller Schneider Patentanwaltsgesellschaft mbH

(57) **Abstract**

A prosthetic femoral component includes an articular component and a bone contacting component. The articular component has a convex articular surface and a concave curved inner surface opposite the convex articular surface. The bone contacting component is least partially insertable within the articular component and has an outer side and an inner side opposite the outer side, the outer side having a convex curved surface received on and complementary to the concave curved inner surface of the articular component, and the inner side being defined by a plurality of adjacent planar segments.

## Description

### CROSS-REFERENCE TO RELATED APPLICATIONS

This application claims the benefit of the filing date of United States Provisional Patent Application No. 63/598,585 filed on November 14, 2023, the disclosure of which is hereby incorporated by reference.

### BACKGROUND OF THE INVENTION

Total knee arthroplasty (TKA) procedures are commonly performed to replace the damaged articulating surfaces of the knee with a prosthetic component. Generally, a surgeon resects a portion of the patient's native bone to create surfaces and/or recesses that accept portions of the implanted prosthetic component. Surgeons typically only resect the amount of bone needed to properly implant the prosthetic component to preserve as much native bone as possible and more generally to avoid causing additional complications with the patient.

In order to cut the bone, various cutting jigs, saws, and, in some cases, robotic devices may be used in combination to perform resections of a distal femur and/or a proximal tibia. Some robotic devices are able to accurately perform cutting motions in areas that are otherwise inaccessible to the reach of humans. With the advancement of robotic devices used during surgeries, improved prosthetic components have also been developed to accommodate the bone cuts made possible by robotic devices.

In some procedures, a combination of robotic cuts and manual cuts are performed to resect and prepare the bone surface. This may be the result of a certain operating center not having sufficient robotic equipment to perform the entire procedure or a loss of power that renders the robotic equipment inoperable, among other reasons. Such procedures often require surgeons to select different prosthetic devices than were initially intended due to the need for manual cuts to prepare the bone, where such manual cuts may be less optimal than robotic cuts. Selection of a non-desired implant may result in longer recovery times, pain surrounding the prosthetic joint, and other similar complications.

Accordingly, a need exists for a modular prosthetic femoral component that is versatile and that may be implanted on a variety of bone surfaces.

### BRIEF SUMMARY OF THE INVENTION

According to one aspect of the present disclosure, a prosthetic femoral component comprises an articular component with a convex articular surface and a concave curved inner surface opposite the convex articular surface; and a bone contacting component at least partially insertable within the articular component, the bone contacting component including an outer side and an inner side opposite the outer side, the outer side having a convex curved surface received on and complementary to the concave curved inner surface of the articular component, and the inner side being defined by a plurality of adjacent planar segments.

In another aspect, the articular component includes a post extending from the concave curved inner surface, the post configured to extend through an aperture of the bone contacting component.

In a different aspect, the post is integrally formed with the articular component.

In a further aspect, the aperture of the bone contacting component is an elongate slot sized such that while the post is received through the slot, the bone contacting component may translate relative to the post.

In another aspect, the plurality of adjacent planar segments that define the inner side of the bone contacting component include five adjacent planar segments.

In yet another aspect, the at least one planar segment of the plurality of adjacent planar segments includes a recessed surface region, the recessed surface region configured to receive bone cement.

In a different aspect, the articular component includes an anterior flange and a posterior flange such that the curvature of the articular component defines a 180° angle between the anterior flange and posterior flange.

In a further aspect, the bone contacting component includes a plurality of pores extending at least partially through the bone contacting component from the inner side to the outer side, the plurality of pores defining a grid pattern over a portion of at least one of the respective inner and outer sides of the bone contacting component.

In another aspect, the bone contacting component includes an anterior component and a posterior component removably attachable to the anterior component.

In a further aspect, the posterior component includes two condylar portions connected by a bridge.

In a different aspect, the anterior component includes one of a protrusion and a recess and the bridge of the posterior component includes the other of the protrusion and the recess, the protrusion configured to be received in the recess to connect the anterior component with the posterior component.

In another aspect, the receiver is a slot and the protrusion is a tab.

In a further aspect, the posterior component is configured to hinge relative to the anterior component when the protrusion is received in the recess.

In another aspect, when the posterior component is connected to the anterior component, the posterior component is lockable in a plurality of angular orientations relative to the anterior component.

In another aspect, a prosthetic femoral component kit comprises: the prosthetic femoral component of claim 1, wherein the bone contacting component is a first bone contacting component; and a second bone contacting component that is at least partially insertable within the articular component, the second bone contacting component including a second outer side and a second inner side opposite the second outer side, the second outer side having a convex curved surface received on and complementary to the concave curved inner surface of the articular component, and the second inner side being defined by a second plurality of adjacent planar segments different from the plurality of adjacent planar segments of the first bone contacting component.

According to another aspect of the present disclosure, a method of assembling components of a modular femoral prosthetic assembly, the method comprises: inserting a post of an articular component through a slot of a bone contacting component until a concave curved inner surface of the articular component is adjacent to a convex curved outer surface of the bone contacting component; and rotating the articular component about an axis of rotation through a center of a radius of curvature of the articular component such that the post translates along at least a portion of a length of the slot.

In another aspect, the method further comprises attaching the post to the concave curved inner surface of the articular component before the inserting step.

In a different aspect, the attaching step includes rotating the post to lock the post to the articular component.

In another aspect, the rotating step further includes rotating the articular component until the post contacts an end of an elongate dimension of the slot.

In a further aspect, prior to the inserting step, the method comprises rotating the articular component to a first rotational position relative to the bone contacting component so that the articular component is insertable over the bone contacting component, the first rotational position being different from a second rotational position of the articular component after the rotating step, wherein the inserting step is not performable in the second rotational position.

In a different aspect, the method further comprises inserting a second post of the articular component through a first end of a second slot of the bone contacting component.

According to another aspect of the present disclosure, a method of assembling components of a modular femoral prosthetic assembly, the method comprises: inserting an anterior component of a bone contacting component and a posterior component of a bone contacting component onto a concave curved inner surface of an articular component; prior or subsequent to the inserting step, connecting the anterior component to the posterior component so that the anterior component and the posterior component are hingedly connected; and adjusting a position of the connected anterior and posterior components along the concave curved inner surface of the articular component while inserted on the concave curved inner surface to define an inner surface of the bone contacting component for receipt on a bone, the inner surface of the bone contacting component being opposite an outer surface of the bone contacting component facing the articular component.

In another aspect, the connecting step further comprises inserting a locking tab of the anterior component into a slot of the posterior component.

In a further aspect, the method further comprises locking the angular orientation of the anterior component relative to the posterior component.

In a different aspect, the inserting step occurs before the connecting step.

According to another aspect of the present disclosure, a femoral implant kit comprises: a first bone contacting component including a first inner surface having a first plurality of adj acent planar segments and a first outer surface opposite the first inner surface; a second bone contacting component including a second inner surface having a second plurality of adjacent planar segments and a second outer surface opposite the second inner surface, the second inner surface being different from the first inner surface; and an articular component including an inner curved surface with a post thereon; wherein the first outer surface and the second outer surface are complementary to the inner curved surface of the articular component and the first and second bone contacting components are configured to be received in the articular component.

In another aspect, the first and second bone contacting components include respective first and second internal openings therethrough such that both the first and second bone contacting components are independently receivable on the inner curved surface of the articular component by passing the internal opening over the post, the internal opening having a shape such that the first or second bone contacting component is rotatable relative to the articular component while the outer surface of the first bone contacting component is adjacent to the inner surface of the articular component.

In a different aspect, the first bone contacting component further comprises a posterior component and an anterior component such that in an assembled state, the posterior component and the anterior component are connected to define the first bone contacting component.

In another aspect, the second bone contacting component has a thickness different than a thickness of the first bone contacting component.

In a further aspect, the first bone contacting component includes a plurality of pores defined at least partially through the first bone contacting component.

In a different aspect, the kit further comprises a third bone contacting component receivable on the articular component, the third bone contacting component being different from the first and second bone contacting components.

In another aspect, the kit further comprises a second articular component configured to receive the first and second bone contacting components, the second articular component including an opening extending through an anterior portion of the second articular component.

In another aspect, the post is removably attached to the articular component.

According to another aspect of the present disclosure, a prosthetic femoral component comprises: an articular component with an articular surface and an inner surface opposite the articular surface; and a bone contacting component receivable in the articular component, the bone contacting component comprising: a posterior component including a first condylar portion, a second condylar portion and a bridge connecting the first and second condylar portions, the bridge having a first engagement feature; and an anterior component including a second engagement feature engageable to the first engagement feature, wherein a first outer surface of the posterior component is insertable onto the inner surface of the articular component and a second outer surface of the anterior component is insertable onto the inner surface of the articular component, wherein the posterior component and the anterior component are insertable onto the inner surface of the articular component independently or together, and wherein the posterior component and the anterior component are connected through engagement of the first engagement feature with the second engagement feature.

In a different aspect, the posterior component is engaged to the anterior component, the anterior component is pivotable relative to the posterior component.

In another aspect, the anterior component is lockable to the posterior component at different angular orientations relative to the posterior component.

In a further aspect, the first engagement feature is a slot and the second engagement feature is a tab insertable within the slot.

In another aspect, the first engagement feature is a tab and the second engagement feature is a slot, the tab insertable within the slot.

In a different aspect, the inner surface of the articular component is curved.

In a different aspect, the anterior component further includes a third engagement feature configured to engage with a fourth engagement feature of the articular component.

In a further aspect, the third engagement feature is a hole and the fourth engagement feature is a post insertable within the hole.

In another aspect, the third engagement feature is a tab and the fourth engagement feature is a slot.

In a different aspect, a friction fit is defined between the engagement of the tab and the slot.

### BRIEF DESCRIPTION OF THE DRAWINGS

A more complete appreciation of the subject matter of the present disclosure and the various advantages thereof can be realized by reference to the following detailed description, in which reference is made to the following accompanying drawings:
FIG. 1A is a perspective view of a bone contacting component according to one embodiment of the present disclosure;
FIG. 1B is a side view of the bone contacting component of FIG. 1A;
FIG. 1C is a front view of the bone contacting component of FIG. 1A;
FIG. 1D is a bottom view of the bone contacting component of FIG. 1A;
FIG. 2A is a perspective view of an articular component according to one embodiment of the present disclosure;
FIG. 2B is a side view of the articular component of FIG. 2A;
FIG. 2C is a front view of the articular component of FIG. 2A;
FIG. 2D is a bottom view of the articular component of FIG. 2A;
FIG. 3A is a perspective view of a femoral component assembly including the bone contacting component of FIG. 1A assembled with the articular component of FIG. 2A;
FIG. 3B is an exploded perspective view of the assembly of FIG. 3A;
FIG. 4A is a perspective view of a bone contacting component according to another embodiment of the present disclosure;
FIG. 4B is a side view of the bone contacting component of FIG. 4A;
FIG. 4C is a front view of the bone contacting component of FIG. 4A;
FIG. 4D is a bottom view of the bone contacting component of FIG. 4A;
FIG. 5A is a perspective view of a bone contacting component assembly including the bone contacting component of FIG. 4A assembled with the articular component of FIG. 2A;
FIG. 5B is an exploded perspective view of the assembly of FIG. 5A;
FIG. 6A is a perspective view of a bone contacting component according to another embodiment of the present disclosure;
FIG. 6B is a side view of the bone contacting component of FIG. 6A;
FIG. 6C is a front view of the bone contacting component of FIG. 6A;
FIG. 6D is a bottom view of the bone contacting component of FIG. 6A;
FIG. 7A is a perspective view of a bone contacting component according to another embodiment of the present disclosure;
FIG. 7B is a back view of the bone contacting component of FIG. 7A;
FIG. 7C is a perspective view of an articular component according to another embodiment of the present disclosure;
FIG. 7D is an exploded perspective view of a femoral component assembly including the bone contacting component of FIG. 7A assembled with the articular component of FIG. 7C;
FIG. 8A is a bottom view of a bone contacting component according to another embodiment of the present disclosure;
FIG. 8B is a partially exploded perspective view of a femoral component assembly including the bone contacting component of FIG. 8A assembled with the articular component of FIG. 2A;
FIG. 8C is a perspective view of the assembly of FIG. 8B;
FIG. 8D is a side view of the assembly of FIG. 8B in which the bone contacting component is iteratively illustrated;
FIGS. 9A-9C are perspective views of a modular bone contacting component according to another embodiment of the present disclosure;
FIGS. 9D-9I are various view of a femoral component assembly including the bone contacting component of FIG. 9C assembled with the articular component of FIG. 2A;
FIG. 10A-10C are various views of a bone contacting component according to another embodiment of the present disclosure;
FIG. 10D is an exploded perspective view of a femoral component assembly including the bone contacting component of FIG. 10A-10C and an articular component; and
FIG. 11 is a perspective view of a modular femoral component assembly including modular posts.

### DETAILED DESCRIPTION

Reference will now be made in detail to the various embodiments of the present disclosure illustrated in the accompanying drawings. Wherever possible, the same or like reference numbers will be used throughout the drawings to refer to the same or like features within a different series of numbers (e.g., 100-series, 200-series, etc.). It should be noted that the drawings are in simplified form and are not drawn to precise scale. As used herein, the terms "about," "approximately," "generally," and "substantially" are intended to mean that slight deviations from absolute are included within the scope of the term so modified. To aid the Patent Office and any readers of any patent issued on this application in interpreting the claims appended hereto, Applicant notes that it does not intend any of the appended claims or claim elements to invoke 35 U.S.C. § 112(f) unless the words "means for" or "step for" are explicitly used in the particular claim.

In describing preferred embodiments of the disclosure, reference will be made to directional nomenclature used in describing the human body. It is noted that this nomenclature is used only for convenience and that it is not intended to be limiting with respect to the scope of the present disclosure. As used herein, when referring to bones or other parts of the body, the term "anterior" means toward the front part of the body or the face, and the term "posterior" means toward the back of the body. The term "medial" means toward the midline of the body, and the term "lateral" means away from the midline of the body. The term "proximal" means closer to the heart, and the term "distal" means more distant from the heart.

In one aspect, the present disclosure relates to a femoral implant assembly. Such assembly may include two or more components, with an articular component that defines an articular surface and bone contacting component that is received in the articular component on one side and mates with a bone surface on an opposite side. Such a femoral implant assembly allows an operator to have the option of using a single articular component that is compatible with multiple inner components depending on the particular application. Throughout the disclosure, femoral implant assembly may also be referred to as femoral component assembly. For example, an inner component can be selected that is best suited for an existing bone surface shape or based on what is a potential option given an existing bone condition. As an example, an inner component can be selected that corresponds to planar manual cuts made by an operator if power is lost during a procedure such that curved robotic cuts cannot be utilized in their entirety of the procedure. The inner component, selected from among a group of inner components, can be assembled with a single articular component that functions as a universal articular component. Furthermore, if a procedure implements robotic devices to cut the bone and an operator supplements additional manual cuts after use of the robotic device, an operator may then swap the previous modular inner component with a new modular inner component that is best married with the modified bone surface such that a new assembly is not required.

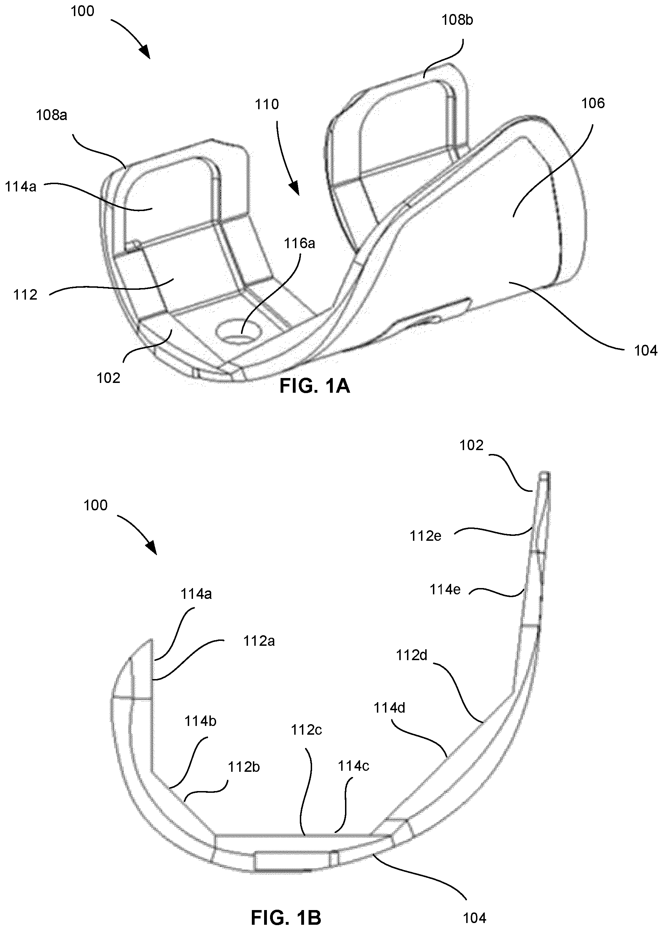

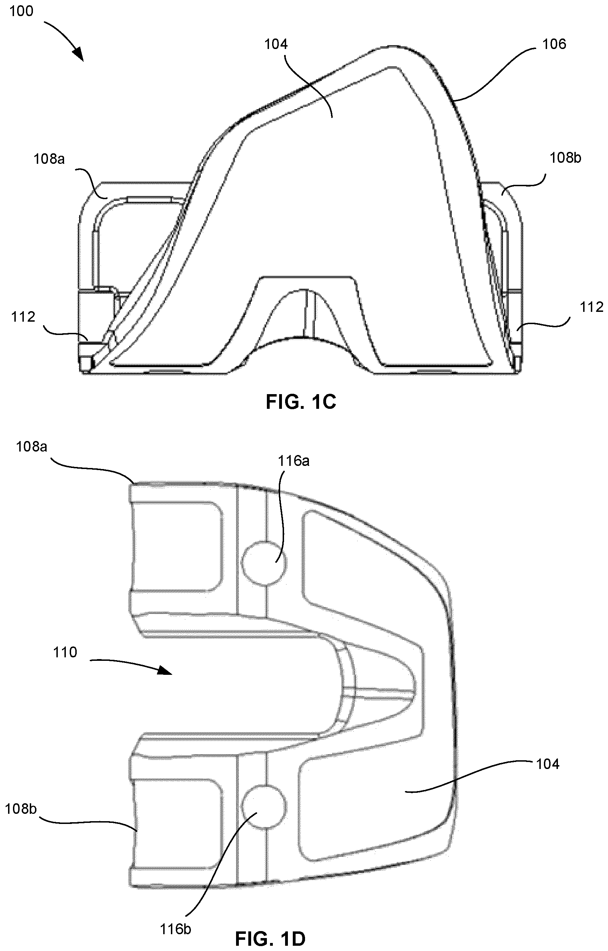

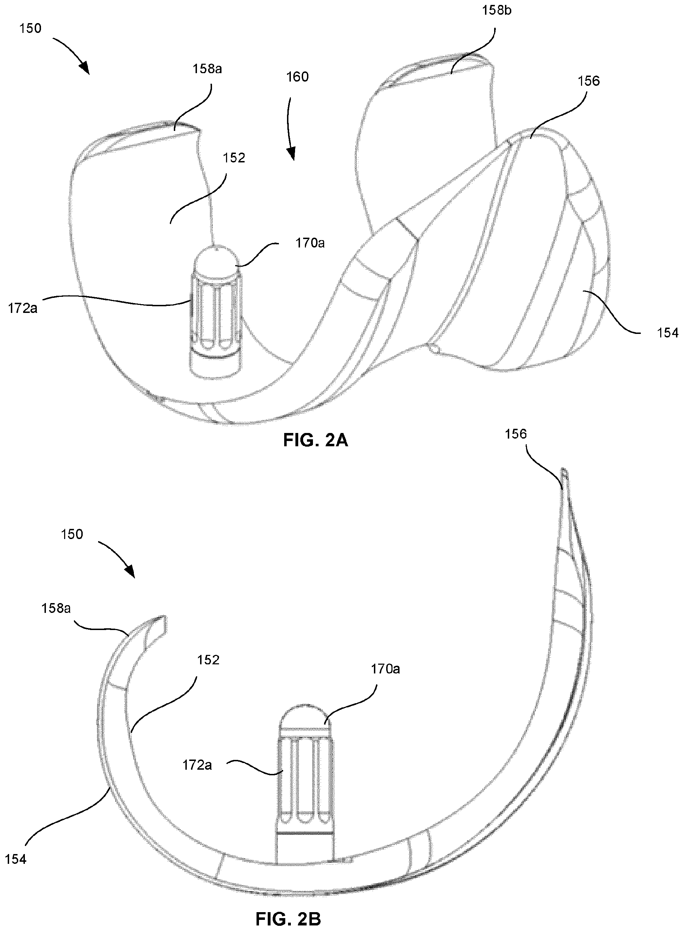

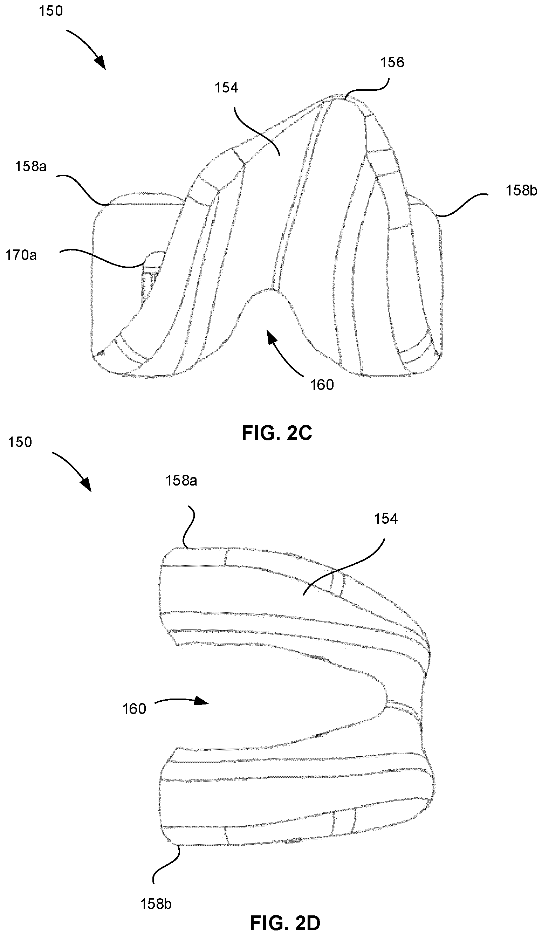

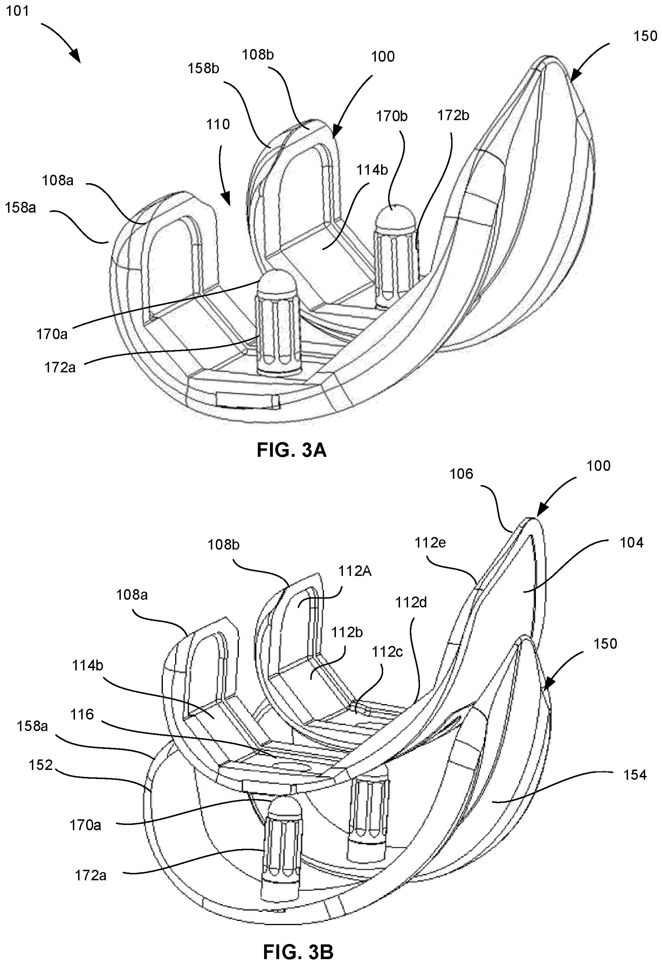

In one embodiment, a femoral implant assembly may include an articular component 150, shown, for example, in FIGS. 2A-2D, and a bone-contacting femoral component 100, shown in FIGS. 1A-1D. It should be appreciated that bone-contacting femoral component 100 can be nested within an inner concave side of articular component 150 to form a femoral implant assembly 101, shown, for example, in FIG. 3A. Throughout the disclosure, the bone-contacting femoral component may also be referred to as inner component.

Bone contacting component 100 includes a concave inner surface 102 and a convex outer surface 104. Throughout the disclosure, the concave inner surface and convex outer surface may also be referred to as the inner and outer sides or surfaces. Each of the inner and outer surfaces 102, 104 extend between an anterior portion 106 and condylar portions 108a, 108b. A groove 110 is defined between condylar portions 108a, 108b. Convex outer surface 104 of each condylar portion is configured to at least partially contact one of the medial and lateral condyles of the bone while inner surface 102 is configured to contact a distal end of a patient's femur.

With continued reference to bone contacting component 100, inner surface 102 is configured to contact and adhere to a portion of the femur that has been resected. In some examples, resection of the femur may be performed with robotically controlled cutting tools. The assembly 101 of the present embodiment is adapted for implantation in cases where the resection of the distal femur involves a plurality of planar cuts. For bone surfaces prepared in such manner, inner surface 102 defines several adjacent planar surfaces 112 configured to contact a resected portion of bone such that the respective planar bone portions contact the planar implant portions. Throughout the disclosure, the adjacent planar surfaces may also be referred to as adjacent planar segments. As shown in FIG. 1B, inner surface 102 of femoral component 100 includes five planar surfaces 112a-112e. Each planar surfaces 112a-e has unique dimensions. For example, planar surface 112e of the anterior portion 106 may be longer than the planar surfaces 112a within the condylar portions 108a, 108b. Furthermore, although five planar surfaces 112 are illustrated, it is envisioned that any size and/or quantity of planar surfaces may be formed into inner surface 102 of bone contacting component 100 to correspond to various resections of a patient's femur performed during an operation. And, with the modular nature of the design, the bone contacting component may be one of several or many bone contacting components each with different planar surfaces on an inner surface of the component. Inner surface 102 further defines various pockets 114a-e that can be filled with bone cement to aid in adhering femoral component 100 to bone. Pockets 114a-e can be defined as a recess in each planar surface 112a-112e, and thus five pockets 114a-114e correspond to the respective five adjacent planar surfaces 112a-112e and are illustrated as such in FIG. 1B. Throughout the disclosure, recess may also be referred to as recessed surface region. The pockets may additionally act as areas for allowing porous ingrowth of the surrounding bone to the implant.

As to the outer surface of component 100, outer surface 104 is convex and defines a generally curved surface. In the depicted embodiment, outer surface 104 is smooth along a length extending from a posterior end of component 100 to an anterior end though has some variation in its radius of curvature across such distance. A curvature of outer surface 104 may be designed to best reflect the natural kinematics of the patient, i.e., restore a natural relationship between the femur and tibia over a range of motion between flexion and extension. As illustrated, the curvature of outer surface 104 extends approximately 180° between anterior portion 106 and condylar portions 108a, 108b. In such a configuration, the planar surfaces 112a of the condylar portions 108a, 108b and planar surface 112e of the anterior portion 106 generally oppose each other and extend in parallel or close to parallel planes. Outer surface 104 is substantially smooth and shaped to complement a smooth inner surface of articular component 150. In an alternative embodiment, the outer surface may include pockets adapted for the receipt of bone cement.

As shown in FIG. 1D, holes 116a, 116b may be formed through respective condylar portions 108a, 108b. Throughout the disclosure the holes may be referred to as apertures. Each hole 116a, 116b may be sized to receive a peg on or otherwise attachable to articular component 150 and thus aid in attaching bone contacting component 100 to an articular component 150. These features are described in greater detail below.

FIGS. 2A-2D illustrate an articular femoral component, also referred to as an articular component 150, according to one embodiment of the present disclosure. It is appreciated that articular component 150 is compatible with bone contacting component 100 to form femoral component assembly 101, illustrated in FIG. 3A-3B, and is further compatible with other bone contacting components described herein. As such, articular component 150 is in one respect a "universal" articular component that provides freedom to an operator to choose a compatible inner component that best suited for implantation on a specific patient bone in a knee surgery. Further, it is appreciated that articular component 150 is compatible with a femoral bone without an inner component; thus, articular component 150 can be used in procedures where larger amounts of native bone are preserved. One example of such a procedure would be where a robot is used to cut a rounded end surface on the distal femur maximizing preservation of the native bone. In this manner, articular component 150 may be a standalone implant in some use cases.

Articular component 150 includes a concave inner surface 152 and a convex outer surface 154. Throughout the disclosure, the concave inner surface and the convex outer surface may be referred to as the concave curved inner surface or the convex articular surface. Each of the respective inner and outer surfaces 152 and 154 extend between anterior portion 156 and condylar portions 158a, 158b. Articular component 150 is further characterized by a groove 160 defined by the inside edges of condylar portions 158a, 158b. Further, articular component 150 may have some asymmetry based on the representative characteristics of a natural distal femur.

Convex outer surface 104 is configured to contact a tibia in a post-operative condition when component 150 is implanted in a patient. Concave inner surface 152 of articular component 150 either receives an outer surface of inner component 100 or another similar component, or is directly implanted onto a prepared bone surface of a distal femur. As used in part of an assembly 101, articular component 150 is configured to contact the curved outer surface 104 of bone contacting component 100. As such, the varying radius of curvature of outer surface 154 along an anterior-posterior direction of component 150 is the same or substantially similar to the radius of curvature of outer surface 104 of bone contacting component 100 and is preferably smooth to aid in placement of the articular component 150 relative to the bone contacting component 100. Such a smooth inner surface 152 further allows articular component 150 to be compatible with a variety of bone contacting components, and thus allows an operator to choose between such bone contacting components during a procedure depending on the condition of, including any cuts made to a bone receiving the implant. Outer surface 154 is also curved similarly to inner surface 152, and thus may have a similarly varying radius of curvature along its length so that a thickness of component 150 does not vary dramatically from an anterior end to a posterior end of the component. Outer surface 154 also includes a contact surface portion that defines a line of contact between articular component 150 and a native tibia or a tibial implant of the knee joint. As mentioned above, the inner surface 152 may directly contact the bone without an associated bone contacting component. In such scenarios, the native bone is prepared to complement the articular component shape 150 to ensure a proper fit.

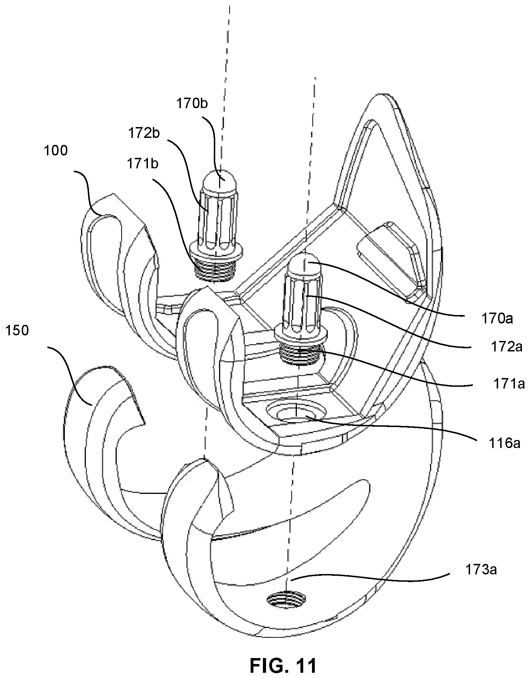

With continued reference to articular component 150, the articular component 150 also includes a pair of posts (one post 170a is shown on condylar portion 158a in FIG. 2A). The posts extend radially inward from respective condylar portions 158a, 158b. While the description below references post 170a specifically, it should be appreciated that the characteristics of the described post apply equally to the second post 170b. Post 170a extends radially inward from inner surface 152 and is sized to extend through hole 116a of bone contacting component 100. Post 170a includes a plurality of longitudinally oriented and spaced apart ridges 172a configured to improve gripping and engagement to the distal femoral bone and to allow ingrowth of the bone into the implant. These characteristics improve the overall securement of the implant to the bone. Post 170a also includes a dome-shaped free end to aid in guiding the post 170a through hole 116a. It is appreciated that post 170a may be integrally formed with articular component 150, or may be a separate component that is attached via a threaded connection, fasteners, adhesives, and the like. One example embodiment where the posts are a separate and distinct component from the bone contacting component is illustrated in FIG. 11, where first and second posts 170a, 170b include a threaded region 171a, 171b that threads into a threaded hole (one hole 173a shown in FIG. 11), thereby securing the posts 170a, 170b to articular component 150.

FIGS. 3A-3B illustrate femoral component assembly 101 including both bone contacting component 100 and articular component 150. In particular, FIG. 3A is an assembled perspective view of assembly 101 and FIG. 3B is an exploded perspective view of assembly 101. To assemble bone contacting component 100 and articular component 150, posts 170a, 170b of articular component 150 are aligned with respective holes 116a, 116ba, of bone contacting component 100. Articular component 150 may then be moved toward bone contacting component, or vice versa, until the outer surface 104 of inner component 100 contacts or substantially contacts inner surface 152 of articular component 150. Bone cement may be placed between such surfaces to aid in the adhesion of the two surfaces together.

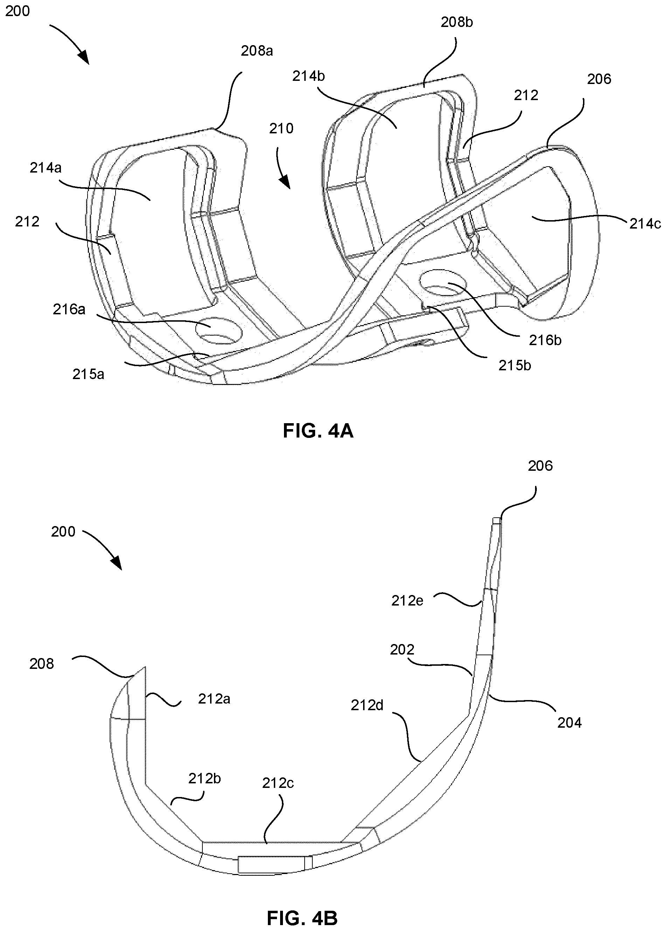

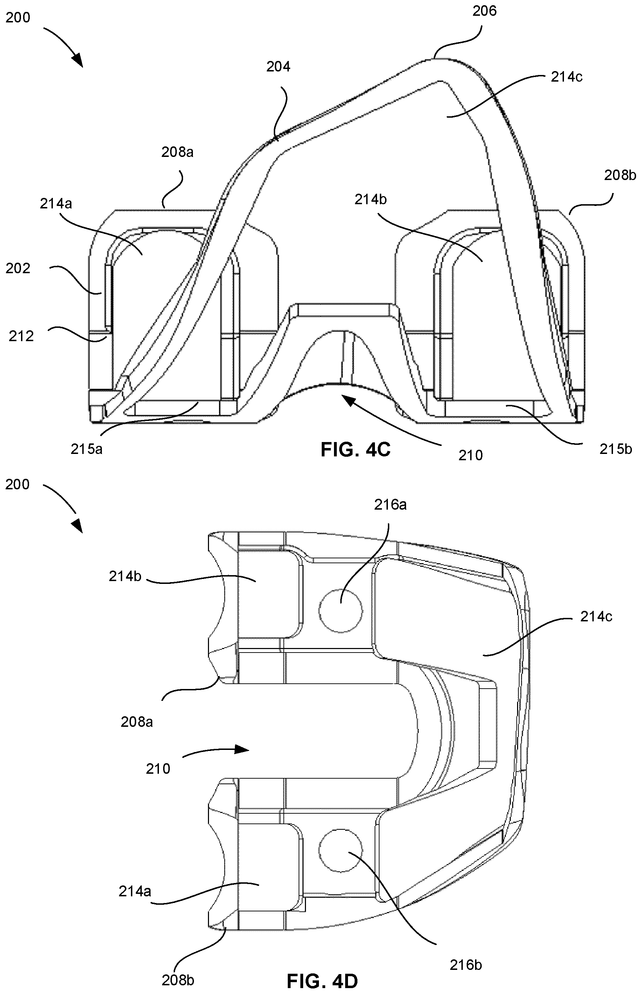

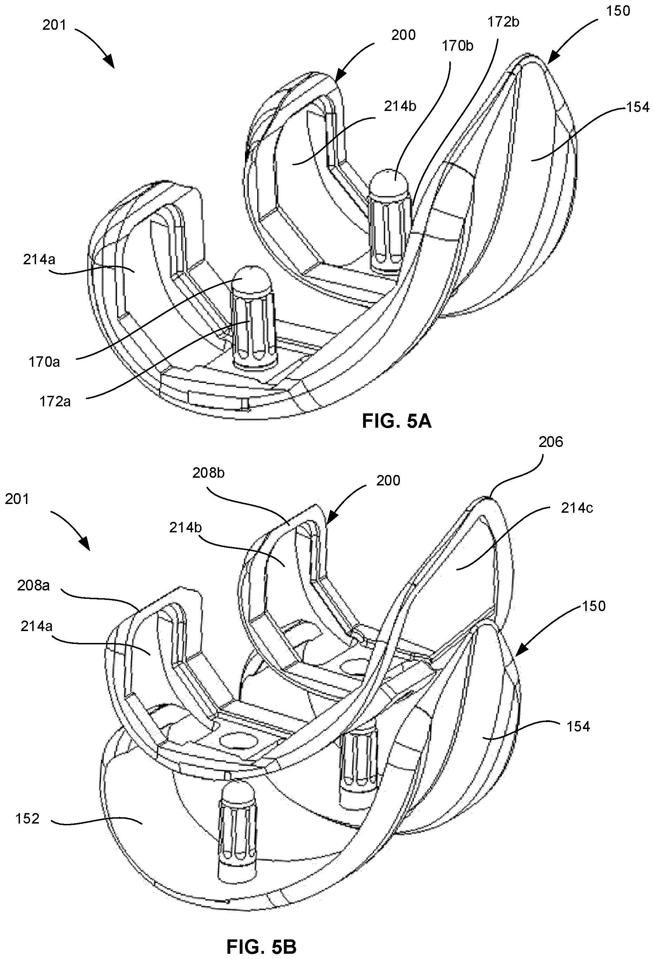

FIGS. 5A-5B illustrate another embodiment of femoral component assembly 201. Assembly 201 includes a bone contacting component 200, shown in FIGs. 4A-4D, and articular component 150, described above and shown in FIGs. 2A-2D. Specifically, FIG. 5A is an assembled perspective view of assembly 201 and FIG. 5B is an exploded perspective view of assembly 201.

With reference to bone contacting component 200 in particular, it should be appreciated that bone contacting component 200 is compatible with articular component 150 to form femoral component assembly 201. In this embodiment, bone contacting component 200 is similar to bone contacting component 100, and therefore elements in the 200-series of reference numerals refer to like elements within the 100-series of reference numerals. Bone contacting component 200 includes a concave inner surface 202 and a convex outer surface 204. Each of the inner and outer surfaces 202, 204 extend between an anterior portion 206 and condylar portions 208a, 208b, the condylar portions 208a, 208b separated by a groove 210.

Inner surface 202, as shown in FIGs. 4A-4B, for example, is configured to contact a distal end of a patient's femur. Particularly, each of the planar surfaces 212a-e defined within inner surface 202 are configured to at least partially contact a planar cut surface of bone. Each of planar surfaces 212a-e has unique dimensions. For example, planar surface 212e of the anterior portion 206 may be longer than the planar surfaces 212a within the condylar portions 208a, 208b. Furthermore, although five planar surfaces 212a-e are illustrated, it is envisioned that any size and/or quantity of planar surfaces may be part of a design of inner surface 202 of bone contacting component 200. Outer surface 204 is convex and defines a curved surface curved similarly to inner surface 152, and thus may have a similarly varying radius of curvature along its length so that a thickness of component 200 does not vary dramatically from an anterior end to a posterior end of the component. Outer surface 204 is substantially smooth and defines a plurality of openings for allowing bone ingrowth into the implant, and for receiving bone cement. Specifically, two condylar portion openings 214a, 214b are defined within respective condylar portions 208a, 208b, and one anterior portion opening 214c is defined within anterior portion 206. In variations of component 200, a size of openings 214a-c relative to overall dimensions of component 200 may vary relative to that shown. Put another way, the openings may be larger or smaller. Such variations in the design may be adopted to suit particular bone conditions and desired bone ingrowth. As shown, the size and shape of openings 214a-c generally correlate to the size of the corresponding posterior portion 208 and anterior portion 206 by having rim portions with relatively narrow width dimensions to define such openings. For example, a rim, defined in each of planar surfaces 212a-e may have a minimum width of 3mm around the perimeter of the openings 214a-c. In some applications that would benefit from maximizing opening sizes, such as applications that require more bone ingrowth, bone contacting component 200 may have a series of openings defined by respective peripheral rims that are 3mm wide around a majority of such periphery. Likewise, for applications with smaller openings, such as applications that require less bone ingrowth, a larger rim width of 4-5mm may be implemented. Openings 214a, 214b are separated from opening 214c by solid portions 215a, 215b, as shown in FIG. 4A. Holes 216a, 216b are formed through planar surfaces 212 of the respective condylar portions 208a, 208b. Each hole 216a, 216b aids in attaching bone contacting component 200 to articular component 150 or to other femoral components described herein by receiving a post of such articular components.

To assemble bone contacting component 200 and articular component 150 into assembly 201, posts 170a, 170b of articular component 150 are aligned with respective holes 216a, 216b of bone contacting component 200, as shown in FIG. 5B. Articular component 150 may then be moved toward bone contacting component, or vice versa, until the outer surface 204 of inner component 200 contacts or substantially contacts inner surface 152 of articular component 150, as shown in FIG. 5A. Bone cement may be placed between such surfaces to aid in the adhesion of the two surfaces together.

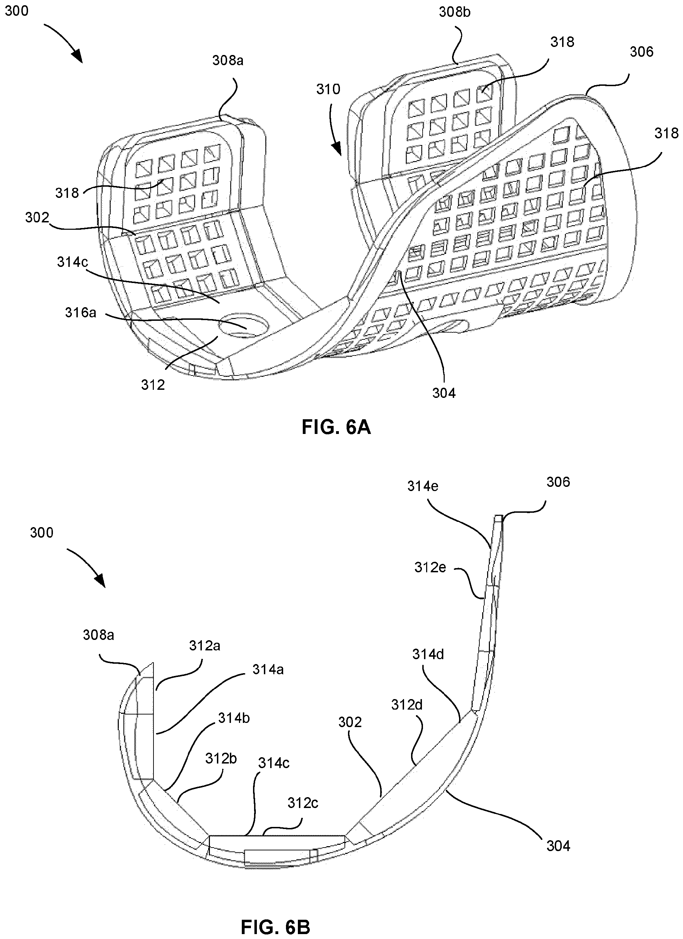

In another embodiment, a femoral component assembly includes a bone contacting component 300, as shown in FIGs. 6A-6D, and an articular component, such as articular component 150, shown in FIGs. 2A-2D. In this manner, it should be appreciated that bone contacting component 300 is compatible with articular component 150 to form a femoral component assembly. In this embodiment, bone contacting component 300 is similar to bone contacting component 100, and therefore elements in the 300-series of reference numerals refer to like elements within the 100-series of reference numerals. Bone contacting component 300 includes a concave inner surface 302 and a convex outer surface 304. Each of the inner and outer surfaces 302, 304 extend between an anterior portion 306 and condylar portions 308a, 308b, the condylar portions 308a, 308b separated by a groove 310.

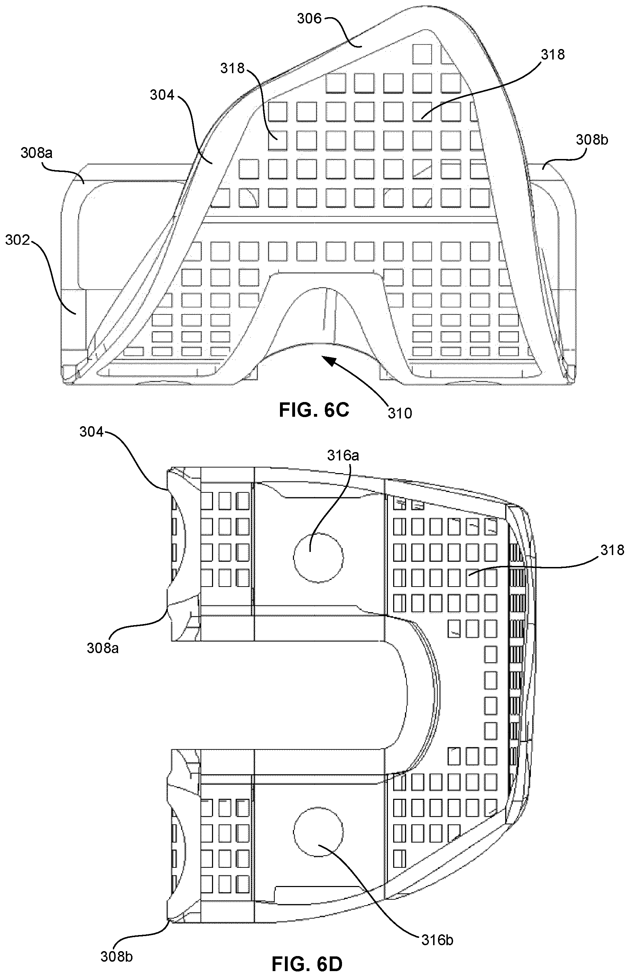

With continued reference to bone contacting component 300, inner surface 302 is configured to contact a distal end of a patient's femur. Particularly, each of the planar surfaces 312a-e defined within inner surface 302 are configured to at least partially contact a planar cut surface of bone. Outer surface 304 is convex and defines a curved surface curved similarly to inner surface 152, and thus may have a similarly varying radius of curvature along its length so that a thickness of component 300 does not vary dramatically from an anterior end to a posterior end of the component. Bone contacting component 300 includes a plurality of pores 318 that define a lattice structure extending partially or entirely through the bone contacting component from the inner surface 302 to the outer surface 304. The plurality of pores are present in regions of component 300 including a majority of planar surfaces 312a, 312b, 312d and 312e, as shown in FIGs. 6A, 6C and 6D. Each pore of the plurality of pores 318 is a square-shaped hole as illustrated in FIG. 6A, or in variations, may be circular or another shape. As shown, plurality of pores 318 define a grid pattern of rows and columns and are generally uniformly spaced across bone contacting component 300. In variations, the pattern defined by the plurality of pores and/or the spacing between individual pores of the plurality of pores may vary. Plurality of pores 318 are configured to allow bone cement to contact both the bone and the bone contacting component without sacrificing structural rigidity that could result from the inclusion of larger openings. Additionally, cement pockets 314a-e are defined within planar surfaces 312a-e, as is illustrated in FIG. 6B, to allow for additional bone cement to interface between the implant and the bone. Holes 316a, 316b are formed through planar surfaces 312c of respective condylar portions 308a, 308b. Each hole 316a, 316b aids in attaching bone contacting component 300 to articular component 150 or to other femoral components described herein by receiving a post of such articular components.

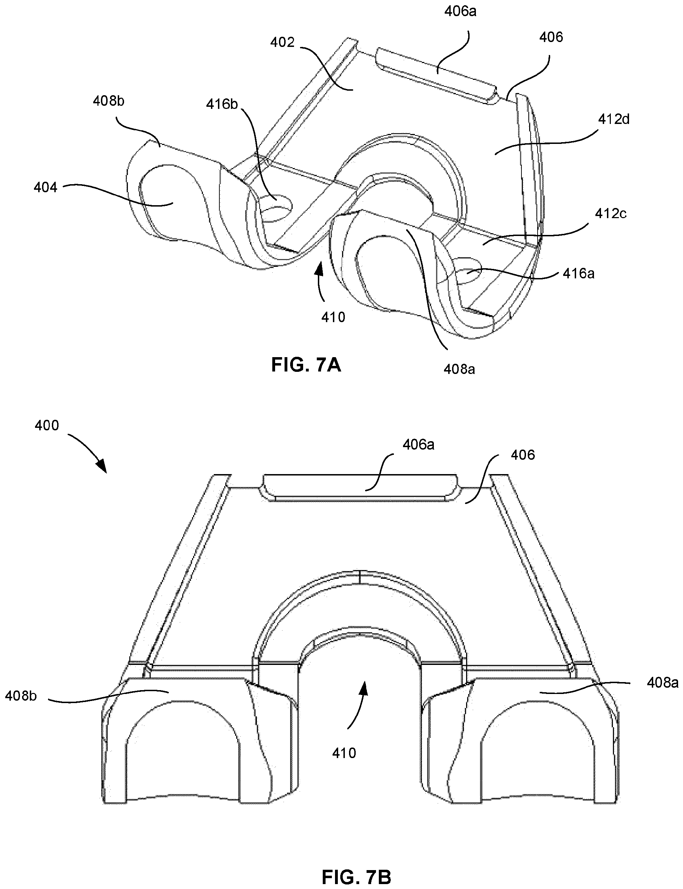

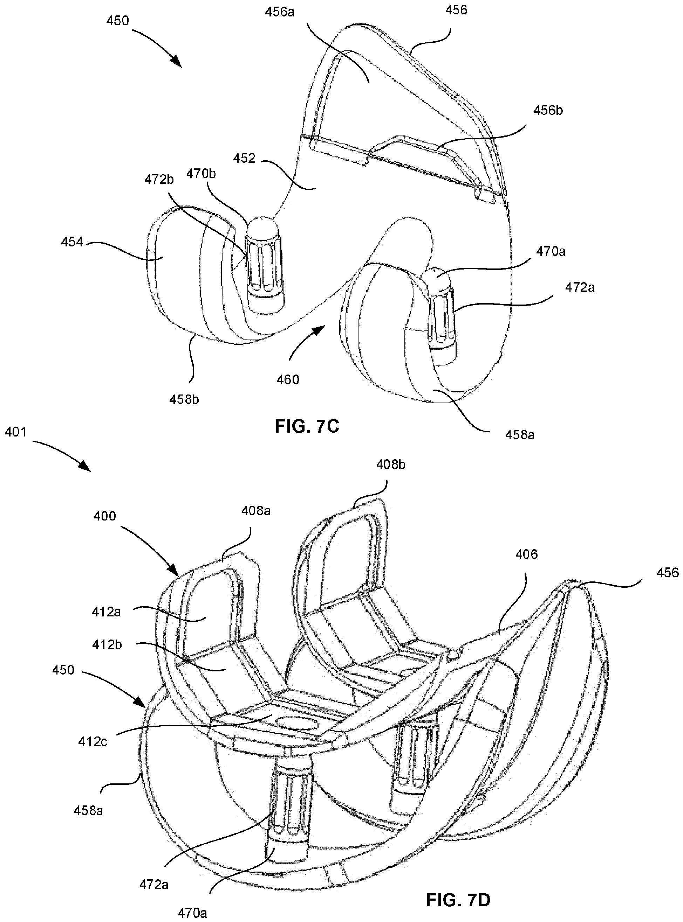

FIG. 7D illustrates yet another embodiment of femoral component assembly 401. Assembly 401 includes a bone contacting component 400, shown in FIGs. 7A-7B, and articular component 450, shown in FIG. 7C. It should be appreciated that while bone contacting component 400 is adapted for use with articular component 450, bone contacting component 400 is also compatible with articular component 150 and other articular components to form a femoral component assembly. In this embodiment, bone contacting component 400 is similar to bone contacting component 100, and therefore elements in the 400-series of reference numerals refer to like elements within the 100-series of reference numerals.

Bone contacting component 400, illustrated in FIGS. 7A-7B, includes a concave inner surface 402 and a convex outer surface 404. Each of the inner and outer surfaces 402, 404 extend between an anterior portion 406 and condylar portions 408a, 408b, the condylar portions 408a, 408b separated by a groove 410. Anterior portion 406 includes a lip 406a configured to enhance engagement with the bone receiving the assembly. Lip 406a may also serve as a locking tab for engagement with a complementary pocket 456b or other feature of anterior flange 456a of articular component 450. Bone contacting component 400 may be symmetric, i.e., anterior portion 406 lies along a center point of groove 410 between condylar portions 408a, 408b. Thus, bone contacting component 400 does not "lean" to either medial or lateral side of the implant.

Inner surface 402 of bone contacting component 400 is configured to contact a distal end of a patient's femur. Specifically, each of the planar surfaces 412a-d defined within inner surface 402 and shown in FIGs. 7A and 7D, are configured to at least partially contact respective planar cut surfaces of bone. Each of planar surfaces 412a-d may have unique or similar dimensions. For example, planar surface 412d of the anterior portion 406 may be longer than the planar surfaces 412a within the condylar portions 408a, 408b. Outer surface 404 is convex and defines a curved surface that complements inner surface 152, and thus may have a similarly varying radius of curvature along its length so that a thickness of component 400 does not vary dramatically from an anterior end to a posterior end of the component. Cement pockets, i.e., recessed regions, may be defined within various planar surfaces 412a-d to allow for additional bone cement to interface between component 400 and the bone. Holes 416a, 416b are formed through planar surface 412c of each condylar portion 408a, 408b. Each hole 416a, 416b aids in attaching bone contacting component 400 to articular component 450 or to other femoral components described herein by receiving a post of such articular components.

Articular component 450, shown in FIG. 7C, is compatible with bone contacting component 400 to form femoral component assembly 401, as illustrated in FIG. 7D. Likewise, articular component 450 is compatible with other bone contacting components described herein. Articular component 450 includes a concave inner surface 452 and a convex outer surface 454. Each of the inner and outer surfaces 452 and 454 extend between an anterior portion 456 and condylar portions 458a, 458b. A groove 460 is defined between condylar portions 458a, 458b. Articular component 450 may be generally asymmetric, i.e., anterior portion 456 is not centered between condylar portions 458a, 458b, and may extend toward either the medial or lateral condylar portion 458a, 458b. Anterior portion 456 includes an anterior flange 456a. Anterior flange 456a is a flat planer cut that extends higher than an anterior portion 406 of bone contacting component 400. Thus, while the articular component 450 is asymmetric to align with either a right or left knee, bone contacting component 400 is symmetric about the condylar portions 408a, 408b such that bone contacting component 400 is attachable to both the right and left knees, and may be inserted into either a right or left articular component 450. Notably, anterior flange 456a may flex along an opening or pocket 456b defined within anterior flange 456a to engage the rounded anterior portion of bone. Pocket 456b is configured to receive bone cement or to allow for bone ingrowth. As such, pocket 456b may have a depth of approximately 1-2mm within the thickness of anterior flange 456a, or pocket 456b may extend entirely through the thickness of anterior flange 456a. The size of pocket 456b may vary to suit different applications. For example, surgical applications with a greater need for bone cement and/or ingrowth may warrant a larger pocket.

Inner surface 452 is configured to contact the curved outer surface 404 of bone contacting component 400. As such, the radius of curvature of outer surface 454 is the same or substantially similar to the radius of curvature of outer surface 404 and is preferably smooth to aid in positioning of the articular component 450 relative to the bone contacting component 400. Outer surface 454 is curved similarly to inner surface 452, and thus may have a similarly varying radius of curvature along its length so that a thickness of component 450 does not vary dramatically from an anterior end to a posterior end of the component to inner surface 452, and thus may have the same radius of curvature to define a uniform thickness between the inner and outer surfaces 452, 454. Outer surface 454 may be substantially smooth or may define a track configured to contact a tibial component or a tibia, as applicable. It is further appreciated that the inner surface 452 may directly contact the bone without an associated bone contacting component. In such scenarios, resection of the native bone is prepared to conform to a contour of inner surface 452 to ensure a suitable fit.

With continued reference to articular component 450, the articular component 450 also includes a pair of posts (one post 470a is shown on condylar portion 458a and the other post 470b shown on condylar portion 458b in FIG. 7C). The posts extend radially inward from respective condylar portions 458a, 458b. While the description below references post 470a specifically, it should be appreciated that the characteristics of the described post apply equally to the second post 470b. Post 470a extends radially inward from inner surface 452 and is sized to pass through hole 416a of bone contacting component 400. Post 470a includes a plurality of longitudinally oriented and spaced apart ridges 472a configured to improve gripping and engagement to the distal femoral bone and to allow ingrowth of the bone into the implant. These characteristics improve the overall securement of the implant to the bone. Post 470a also includes a dome-shaped free end to aid in guiding the post 470a through hole 416a. It should be appreciated that post 470a may be integrally formed with articular component 450, or may be a separate component that is attached via a threaded connection, fasteners, adhesives, and the like.

Femoral component assembly 401, including bone contacting component 400 and articular component 450, is shown in FIG. 7D in an exploded perspective view. To assemble bone contacting component 400 and articular component 450, posts 470a, 470b of articular component 450 are aligned with holes 416a, 416b of bone contacting component 400. Articular component 450 may then be moved toward bone contacting component, or vice versa, until the outer surface 404 of inner component 400 contacts or substantially contacts inner surface 452 of articular component 450. Bone cement may be placed between such surfaces to aid in the adhesion of the two surfaces together.

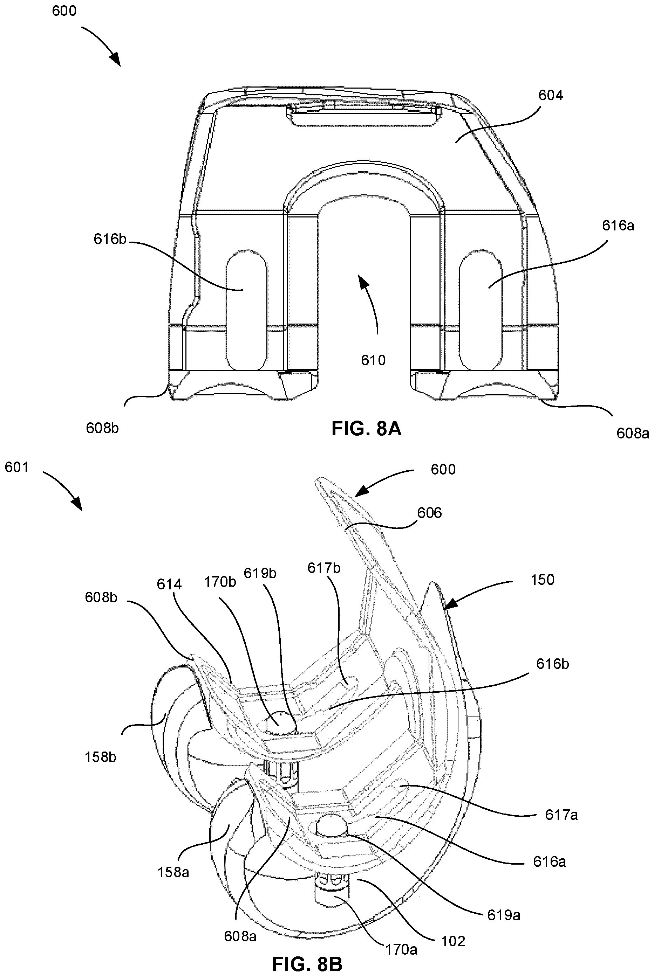

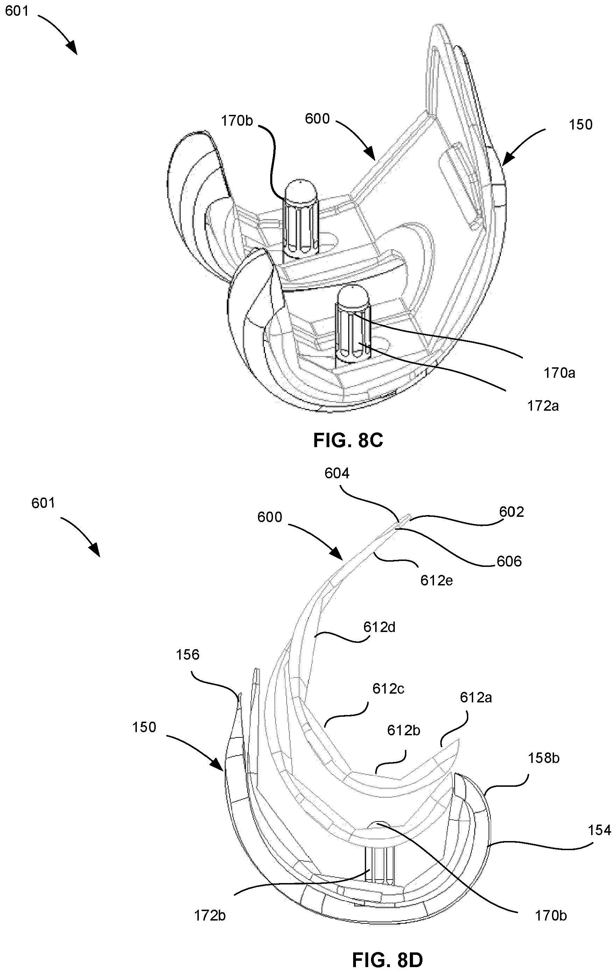

FIGs. 8A-8D illustrates another embodiment of femoral component assembly 601. Assembly 601 includes a bone contacting component 600, shown in FIG. 8A, and articular component 150, described above and shown in FIGs. 8B-8D. In this embodiment, bone contacting component 600 is similar to bone contacting component 100, and therefore elements in the 600-series of reference numerals refer to like elements within the 100-series of reference numerals. Bone contacting component 600, includes a concave inner surface 602 and a convex outer surface 604. Each of the inner and outer surfaces 602, 604 extend between an anterior portion 606 and condylar portions 608a, 608b, the condylar portions 608a, 608b separated by a groove 610. Bone contacting component 600 may be asymmetric, i.e., anterior portion 606 does not lie along a center point of groove 610 between condylar portions 608a, 608b. Thus, bone contacting component 600 is larger on either the medial or lateral side of the implant.

Inner surface 602 of bone contacting component 600 is configured to contact a distal end of a patient's femur. Particularly, each of the planar surfaces 612a-e defined within inner surface 602 are configured to at least partially contact respective planar cut surfaces of the femur bone. Outer surface 604 is convex and defines a curved surface curved similarly to inner surface 152, and thus may have a similarly varying radius of curvature along its length so that a thickness of component 600 does not vary dramatically from an anterior end to a posterior end of the component. Cement pockets 614 may be defined within various planar surfaces 612a-e to allow for additional bone cement to interface between the implant and the bone. Slots 616a, 616b are formed through planar surfaces 612c of respective condylar portions 608a, 608b. Throughout the disclosure, the slots may also be referred to as elongate slots. Each slot 616a, 616b aids in attaching bone contacting component 600 to articular component 150 or to other femoral articular components described herein by receiving posts of such articular components. Slots 616a, 616b are elongated such that each respective slot 616a, 616b extends from a first end 617a, 617b to a second end 619a, 619b. As depicted, each slot 616a, 616b is elongate with rounded ends. With this shape, each slot is sized to slidably receive a post of an articular component. In variations, slots 616a, 616b may be ovular, rectangular, or another shape configured to slidably receive a post. Slots 616a, 616b may further include a locking feature to help lock a post within the slots. Such a locking feature may be a groove, spring, and the like. Bone contacting component 600 preferably includes two slots, each slot positioned entirely through bone contacting component 600 in respective condylar portions 608a, 608b. It is appreciated that while slots 616a, 616b are described in the context of bone contacting component 600, slots may be utilized rather than circular holes for the other bone contacting components disclosed herein.

FIGS. 8B-8D illustrate various views of femoral component assembly 601. Assembly 601 includes bone contacting component 600 and articular component 150. Particularly, FIG. 8B is an exploded perspective view of an assembled perspective view of assembly 601 and FIG. 8B is an exploded perspective view of assembly 601. To assemble bone contacting component 600 and articular component 150, posts 170a, 170b of articular component 150 are aligned with slots 616a, 616b of bone contacting component 600. Articular component 150 may then be moved toward bone contacting component, or vice versa, until the outer surface 604 of inner component 600 contacts or substantially contacts inner surface 152 of articular component 150. Bone cement may be placed between such surfaces to aid in the adhesion of the two surfaces together.

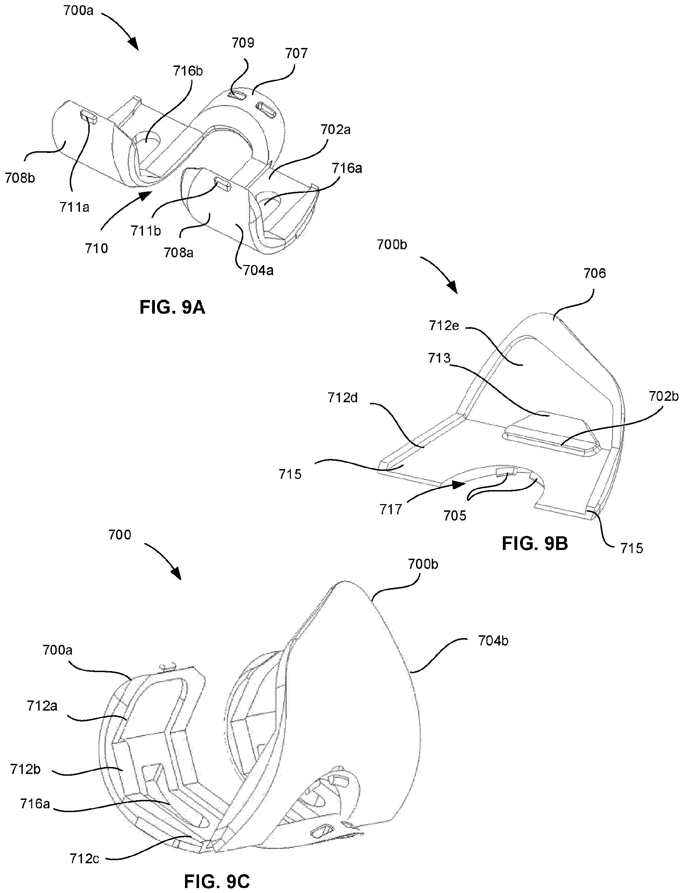

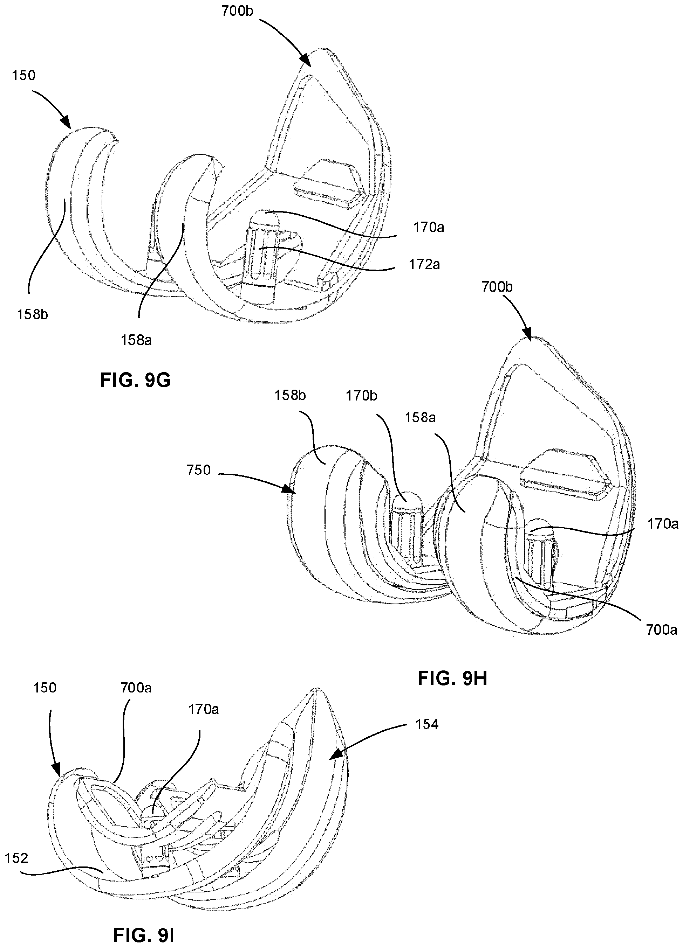

FIGS. 9A-9I illustrate another embodiment of a femoral component assembly. In this embodiment, femoral component assembly 701, illustrated in FIGS. 9A-9C, includes a two-part bone contacting component 700 and an articular component 150.

The two-part bone contacting component 700 includes a posterior component 700a and an anterior component 700b. Posterior component 700a, illustrated in FIG. 9A, includes two condylar portions 708a, 708b connected by a bridge 707. A hole or slot includes two slots 716a, 716b defined through posterior portions 708a, 708b from an inner side 702a to an outer side 704a. Each slot 716a, 716b is positioned through a respective condylar portions 708a, 708b. Bridge 707, as shown, includes two receivers 709. Each receiver 709 may be a slot, threaded opening, or other mechanical connection configured to mate with a complementary mechanism on the anterior component, such as a locking tab. Receiver 709 may also have steps or other features that allow anterior component 700b to be hinged and/or be set to a specific angle relative to posterior component 700a. Each condylar portion 708a, 708b may further include a subtab 711a, 711b configured to engage with an articular component or with a bone. Inner side 702a of posterior component 700a includes a plurality of planar surfaces 712a-c configured to at least partially engage with planar cut surfaces of bone. It should be appreciated that other attachment types may be utilized to attach posterior component 700a to anterior component 700b. For example, a single receiver 709 or three or more receivers 709 may be utilized to receive a locking tab. Likewise, threaded connections, friction fits, and the like may be utilized. In other embodiments, the orientation of the locking tabs and receivers may be switched such that the locking tabs extend from the anterior component 700b and the receivers extend from the posterior component 700a.

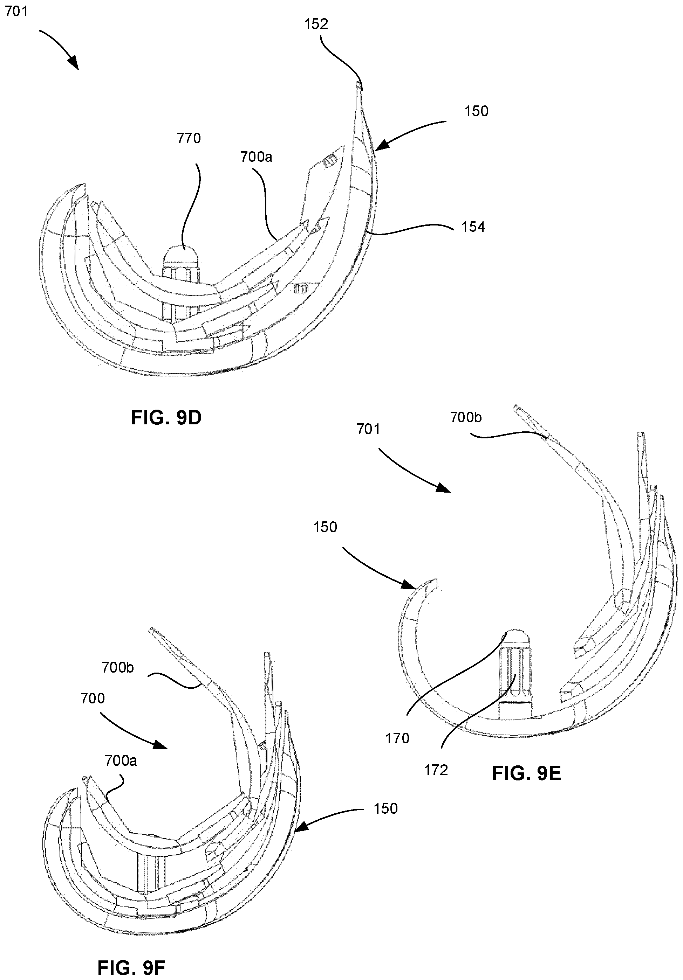

Anterior component 700b, illustrated in FIG. 9B, includes an anterior portion 706 having an inner side 702b and an outer side 704b. A central opening 717 is defined between two legs 715, each leg 715 corresponding to a respective condylar portion 708a, 708b. Two locking tabs 705 extend from the anterior portion 706 and are configured to engage with the receiver 709 of posterior component 700a. Locking tabs 705 may further act as a hinge to allow anterior component 700b to hinge relative to posterior component 700a in order for the implant to be set to a desired location to engage bone. In an assembled configuration, anterior component 700b and posterior component 700a are engaged such that inner side 702b of legs 715 are flush with inner side 702a of condylar portions 708a, 708b. Anterior component 700b further includes a plurality of planar surfaces 712d-e configured to at least partially engage with planar cut surfaces of bone. A protrusion 713 extends outward from an inner side 702b of anterior component 700b and provides increased structural strength and stability to anterior component 700b. Protrusion 713 may be integrally formed with anterior component 700b or may be a separate component attached via adhesives, fasteners, and the like. While FIGs. 9D - 9F show multiple posterior components, multiple anterior components, or multiples of both, it should be appreciated that such depiction is for purposes of illustrating how the components may be received in an articular component, and that an assembly would include one anterior component and one posterior component. A two-piece bone contacting component 700 may be advantageous over a one-piece bone contacting component in that a one-piece bone contacting component may sometimes be too stiff to properly attach to both a bone and an articular component. Additionally, implementing a two-piece bone contacting component 700 may facilitate or otherwise remove obstacles to the assembly process by allowing an operator to manipulate posterior component 700a and anterior component 700b around bone and tissue such that when posterior component 700a and anterior component 700b are attached, the resulting construct can be readily inserted within an articular component. Moreover, the engagement of anterior component 700b and posterior component 700a prevents the respective components from shifting with respect to each other after being positioned within the articular component.

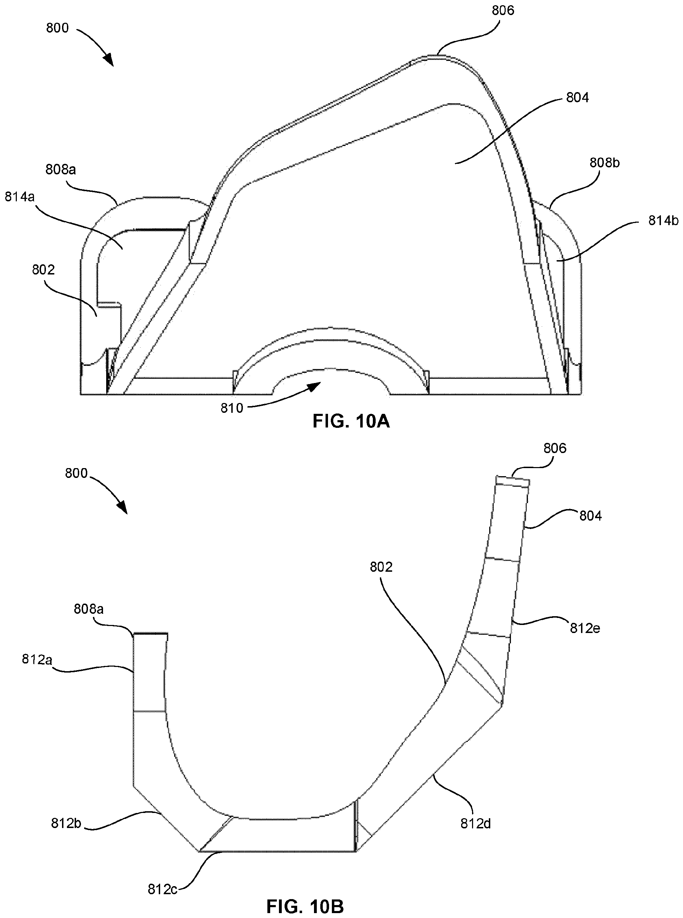

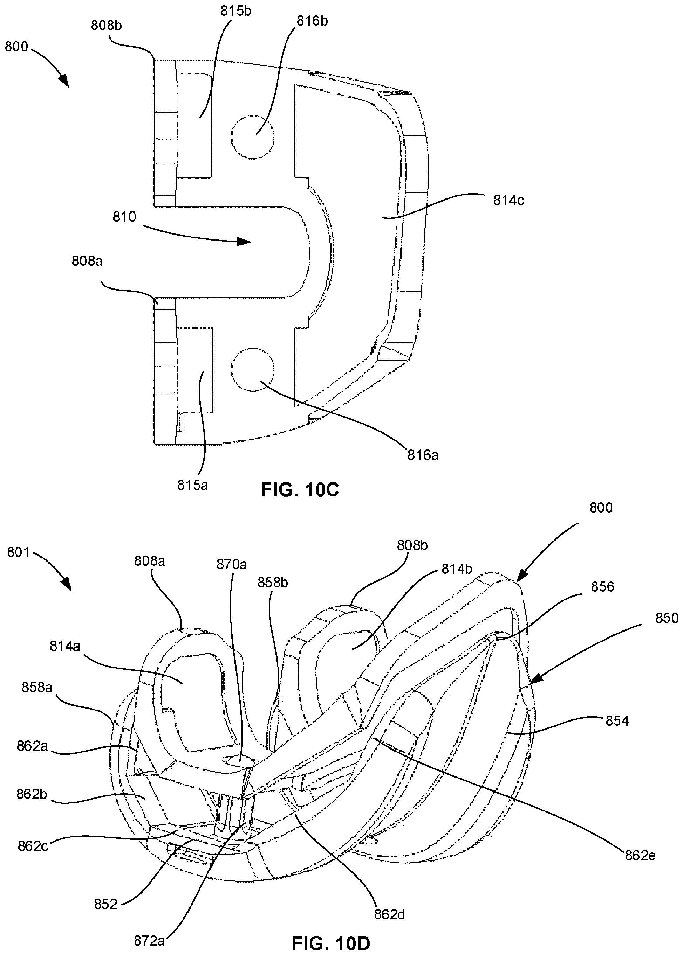

In another embodiment, a femoral component assembly includes a bone contacting component 800, as shown in FIGs. 10A-10D, and an articular component, such as articular component 850, shown in FIG. 10D. In this manner, it should be appreciated that bone contacting component 800 is compatible with articular component 850 to form a femoral component assembly 801. In this embodiment, bone contacting component 800 is similar to bone contacting component 100, and therefore elements in the 800-series of reference numerals refer to like elements within the 100-series of reference numerals. Bone contacting component 800 includes a curved concave inner surface 802 and an outer surface 804 including a plurality of planar faces. Each of the inner and outer surfaces 802, 804 extend between an anterior portion 806 and condylar portions 808a, 808b, the condylar portions 808a, 808b separated by a groove 810.

With continued reference to bone contacting component 800, inner surface 802 is convexly curved to contact a distal end of a patient's femur, and may have a similarly varying radius of curvature along its length so that a thickness of component 800 does not vary dramatically from an anterior end to a posterior end of the component. Inner surface 802 is substantially smooth and defines a plurality of openings for allowing bone ingrowth into the implant. Specifically, two condylar portion openings 814a, 814b are defined within respective condylar portions 808a, 808b, and one anterior portion opening 814c is defined within anterior portion 806. In variations of component 800, a size of openings 814a-c relative to overall dimensions of component 800 may vary relative to that shown. Put another way, the openings may be larger or smaller. Such variations in the design may be adopted to suit particular bone conditions and desired bone ingrowth. As shown, the size and shape of openings 814a-c generally correlates to the size of the corresponding posterior portion 808, 808b and anterior portion 806, and such openings have rim portions with relatively narrow width dimensions to define such openings. For example, a rim, defined in curved inner surface 802 of the bone contacting component 800, may have a minimum width of 3mm around the perimeter of the openings 814a-c. In some applications that would benefit from maximizing opening sizes, such as applications that require more bone ingrowth, bone contacting component 800 may have a series of openings defined by respective peripheral rims that are 3mm wide around a majority of such periphery. Likewise, for applications with smaller openings, such as applications that require less bone ingrowth, a larger rim width of 4-5mm may be implemented. Holes 816a, 816b are formed through flat surfaces 815a, 815b of each condylar portion 808a, 808b. Each hole 816a, 816b aids in attaching bone contacting component 800 to articular component 850 by receiving a post of such an articular component. Outer surface 804 defines a plurality of planar surfaces 812a-e, rather than inner surface 802 defining planar surfaces. Particularly, each of the planar surfaces 812a-e defined within outer surface 804 are configured to contact corresponding planar surfaces of articular component 850.

Articular component 850, shown in FIG. 10D, is compatible with bone contacting component 800 to form femoral component assembly 801, as illustrated in FIG. 10D. Articular component 850 includes a convex outer surface 854 and a concave inner surface 852 including a series of planar surfaces 862a-e. Each of the inner and outer surfaces 852 and 854 extend between an anterior portion 856 and condylar portions 858a, 858b. A groove is defined between condylar portions 858a, 858b. Articular component 850 may be generally asymmetric, i.e., anterior portion 856 is not centered between condylar portions 858a, 858b, and may extend toward either the medial or lateral condylar portion 858a, 858b.

Inner surface 852 is configured to contact the planar outer surface 804 of bone contacting component 800. As such, the dimensions of planar surfaces 862a-e are the same or substantially the same as the dimensions of planar surfaces 812a-e to aid in positioning of the articular component 850 relative to the bone contacting component 800. Outer surface 854 is curved and has a similarly varying radius of curvature along its length so that a thickness of component 850 does not vary dramatically from an anterior end to a posterior end of the component. Outer surface 854 may be substantially smooth or may define a track configured to contact a tibial component or a tibia, as applicable. It is further appreciated that the inner surface 852 may directly contact the bone without an associated bone contacting component. In such scenarios, resection of the native bone is prepared to conform to a contour of inner surface 852 to ensure a suitable fit.

With continued reference to articular component 850, the articular component 850 also includes a pair of posts (one post 870a is shown on condylar portion 858a). The posts extend radially inward from respective condylar portions 858a, 858b. While the description below references post 870a specifically, it should be appreciated that the characteristics of the described post apply equally to the second post. Post 870a extends radially inward from inner surface 852 and is sized to pass through hole 816a of bone contacting component 800. Post 870a includes a plurality of longitudinally oriented and spaced apart ridges 872a configured to improve gripping and engagement to the distal femoral bone and to allow ingrowth of the bone into the implant. These characteristics improve the overall securement of the implant to the bone. Post 870a also includes a dome-shaped free end to aid in guiding the post 870a through hole 816a. It should be appreciated that post 870a may be integrally formed with articular component 850, or may be a separate component that is attached via a threaded connection, fasteners, adhesives, and the like.

Femoral component assembly 801, including bone contacting component 800 and articular component 850, is shown in FIG. 10D in a partially exploded perspective view. To assemble bone contacting component 800 and articular component 850, post of articular component 850 is aligned with holes 816a, 816b of bone contacting component 800. Articular component 850 may then be moved toward bone contacting component, or vice versa, until the outer surface 804 of inner component 800 contacts or substantially contacts inner surface 852 of articular component 850. Bone cement may be placed between such surfaces to aid in the adhesion of the two surfaces together.

The femoral implant components described herein may be made from a variety of materials using a variety of manufacturing methods. For example, the inner bone contacting components and articular components may be constructed of a metal such as aluminum, titanium, or the like. In some examples, such components may be constructed from polyetheretherketone (PEEK) and may be injection molded or formed through other similar methods. The posts attaching the articular component to the bone contacting component and/or the bone may be constructed from a metal or a polymer. Additionally, any of the components described herein may be coated with a porous coating that aids in allowing native bone to grow into the implant and may reduce the amount of bone cement required to secure the implant. Such a porous coating may be manufactured through additive manufacturing with materials such as polyetheretherketone (PEEK) or other similar materials.

In some arrangements, the components described herein may be rigid or have some degree of flexibility. In some such arrangements, the components may be made of a flexible polymer such as, but not limited to, PEEK, polyaryletherketones ("PAEK") and ultrahigh molecular weight polyethylene ("UHMWPE"). The porous structures described herein may be fabricated from, but are not limited to being fabricated from, titanium, titanium alloys, stainless steel, cobalt chrome, tantalum and niobium.

In another aspect, the present disclosure relates to a kit. A kit may include any of the various components described herein in any combination and in any quantity. For example, in some embodiments, each of bone contacting components 100, 200, 300, 400, etc. may be combined in a kit with articular component 150, or variations of articular component 150. In other embodiments, a kit may include multiple sizes of a specific type of bone contacting component, e.g., 100, 200, 300, 400, 600, 700, along with an articular component 150. Sizes may be distinguished by a thickness of the bone contacting component and the position and angle of the planar surfaces on the inner surface, e.g., surfaces 112a-e. For instance, each bone contacting component in the kit may have a different thickness and/or a different inner surface shape, but all having an outer convex surface adapted to be received in the articular component. In any one of the above embodiments, a kit may include two or more articular components. Such articular components may be the same or may be different. In examples where multiple different articular components are included, such articular components may represent different sizes for universal use. In some kits, the articular component of the kit may include posts integrally formed thereon. In other kits, the articular component may be a standalone element separate from the posts. In such cases, posts may be modular and attachable to the articular component. Modular posts of differing lengths and diameters may be included and attached to the articular component to allow an operator the ability to choose the components that best fit a particular application. Further, a kit may also include various insertion instrumentation, such as grippers, jaws, cutting tools, reamers, etc., and other instrumentation used to implant the femoral components. In still further embodiments, any of the contemplated kits may also include other implant components such as tibial components, patellar components, etc.

The contemplated kits allow an operator to have freedom to choose a specific inner component that fits on a prepared distal femur while also complementing and fitting within a single general articular component. This freedom is particularly advantageous in procedures that utilize both robotic bone cuts and manual bone cuts. During a procedure, an operator may assess a patient's bone condition and seek to preserve as much native bone as possible. Where robotic cutting tools are available, the operator may cut a smooth convex shape on the bone to conform to an available articular component as contemplated herein. If the cut cannot be completed, e.g., due to a power failure, or circumstances change and it is determined that the planned smooth convex shaped cut is not expected to yield a stable post-operative structure, then the operator may switch to making planar cuts. In such case, a bone contacting component may be readily selected from a kit that includes multiple bone contacting components, and the appropriate planar cuts may be prepared. Of course, such planar cuts may be prepared through a variety of means, such as through manual operation of tools, and thus may be prepared under a multitude of circumstances and conditions such that the operator will not be prevented from carrying out the procedure in a timely manner.

Another example of a beneficial kit is one in which an operator determines that large amounts of bone cement and/or bone ingrowth are required to secure an implant. With a kit inclusive of components with through openings, an operator may select a bone contacting component having a large number and/or size of through openings, such as inner component 200, that are configured for receiving bone cement and allow native bone to grow into the implant, thereby securing the implant. In other examples, a kit may be well suited for revision procedures, where an extent of bone loss may vary greatly among patients. For such surgical applications, a kit including an array of bone contacting components with a range of thicknesses is advantageous in that a range of patient bone decay conditions may be accommodated with an appropriate bone contacting component from the kit.

In another aspect, the present disclosure relates to methods of manufacturing a femoral implant assembly including one or more of the components thereof. The implantable prosthetic components contemplated by the present disclosure may be manufactured by various methods such as additive manufacturing or various forms of conventional manufacturing. Examples of additive manufacturing techniques that may be utilized include Fused Deposition Modelling ("FDM"), Shape Deposition Manufacturing ("SDM"), Selective Laser Power Processing ("SLPP"), Direct Metal Laser Sintering ("DMLS"), Selective Laser Sintering ("SLS'), Selective Laser Melting ("SLM"), Selecting Heating Sintering ("SHS"), Electron Beam Melting ("EBM"), material jetting, binder jetting, or the like. Additional details of exemplary additive manufacturing methods are described in U.S. Pat. Nos. 7,537,664, 8,590,157, 8,728,387, 9,180,010 and 9,456,901, the disclosures of which are hereby incorporated by reference herein in their entireties. As to conventional manufacture, methods of manufacture may include injection molding, among others.

In another aspect, the present disclosure relates to methods of implanting the femoral assembly and its constituent components. Embodiments of such method are set forth below. For sake of brevity, a general method of implanting femoral component assembly 101 is described. For methods of implantation involving other assemblies, the description indicates any differences that may apply.

An operator may first prepare a distal femur for an implantation procedure. This preparation may entail removing tissue surrounding the patient's knee and performing a series of cuts to the distal femur once access to the bone is created. Because of the modularity of the femoral component 101, an operator may make cuts that result in a medically-optimal implant location, i.e., the operator may assess the bone and determine the condition of native bone and how much of the native bone can be removed. An operator may then select components of the femoral implant system described herein that best correlate to the bone cuts. In one example, a robot may be used to perform a minimal cut leaving a rounded bone surface sized to receive an articular component 150. In another example, planar cuts may be made in accordance with a shape of at least one available bone facing component.

In examples where the bone surface is prepared for receipt of articular component 150, bone cement may be used to secure articular component 150 to the bone surface. In examples where the distal femur is prepared with planar bone cuts, bone contacting component 100 may be attached to the femoral bone using bone cement. Particularly, an operator may apply bone cement to inner side 102 and pockets 114a-e within bone contacting component 100. The operator may then align the condylar portions 108a, 108b with the condyles of the bone and the anterior portion 106 with the anterior portion of the bone. The inner side 102 of femoral bone facing component 100 may then be pressed into the bone such that the bone cement, once cured, adheres the bone contacting component 100 to the bone.

With continued reference to examples of the embodiment with planar cuts to the distal femur, to attach articular component 150 to bone contacting component 100, an operator may first ensure that the concavities of both components are oriented properly, i.e., both concave portions are facing the same direction. The operator may then ensure that the posterior/anterior alignment of the components is proper. To do so, the condylar portions 408a, 408b of the bone contacting component 100 may be aligned with the condylar portions 158a, 158b of articular component 150. To ensure the medial/lateral alignment of the components is proper, an operator may align the longitudinal axis of posts 170a, 170b with the central axis of holes 416a, 416b. In embodiments where posts 170a, 170b is not integral with articular component 150, posts 170a, 170b may first be secured to articular component 150 via threads or other mechanical securement types. Once aligned, articular component 150 can be moved toward bone contacting component 100 such that posts 170a, 170b extend through holes 416a, 416b. Posts 170a, 170b may further be driven into the bone, either through advancement of posts 170a, 170b into predrilled pilot holes and/or through pressing posts 170a, 170b directly into bone by driving such posts through cancellous bone of the distal femur via a press fit. In embodiments where bone contacting component 100 is not utilized, articular component 150 can be pressed directly into bone via posts 170a, 170b without the need to first extend through holes of a bone contacting component. Such a pressing can be accomplished by means as described above.

In the assembly embodiment of FIGS. 8B-8D, articular component 150 can be attached to bone contacting component 600 using similar methods as described for assembly 101, albeit with slots 616a, 616b rather than holes 416a, 416b. Because slots 616a, 616b are elongated, the slots through bone contacting component can translate over respective posts 170a, 170b via an anterior/posterior rolling or sliding motion. In one example, such rolling motion may be accomplished by first extending posts 170a, 170b through first ends 617a, 617b of slots 616a, 616b and then tilting either the inner or articular component such that posts 170a, 170b translate through slots 616a, 616b toward the opposing ends 619a, 619b. If slots 616a, 616b include locking features, an operator can ensure that the posts 170a, 170b are properly engaging the locking features. This process is illustrated in iterations in FIGS. 8B and 8D, which illustrate the bone contacting component 600 being attached to the articular component 150.

In the embodiment of FIGS. 9A-9I, articular component 150 can be attached to bone contacting component 700 using similar methods, albeit with modular bone contacting components. To attach posterior component 700a to anterior component 700b, an operator can align the locking tabs 705 of anterior component 700b with receivers 709 of posterior component 700a. Locking tabs 705 can be pressed into receiver 709 and then the posterior and anterior components can be hinged relative to each other to achieve a desired orientation that mates with a distal end of the femoral bone. The resulting bone contacting component 700, inclusive of posterior and anterior components 700a, 700b, may then be attached to articular component 150. Alternatively, the posterior component 700a may first be attached to articular component 150 and then the anterior component 700b may be attached to the posterior component 700a, in a process shown in part in FIG. 9D. Or, in yet another alternative, the anterior component 700b may be positioned within the articular component 150 and then posterior component 700a may be attached to anterior component 700b, such as is shown in FIGS. 9E and 9F.

It is to be further understood that the disclosure set forth herein includes any possible combinations of the particular features set forth above, whether specifically disclosed herein or not. For example, where a particular feature is disclosed in the context of a particular aspect, arrangement, configuration, or embodiment, that feature can also be used, to the extent possible, in combination with and/or in the context of other particular aspects, arrangements, configurations, and embodiments of the technology, and in the technology generally.

Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative embodiments and that other arrangements may be devised without departing from the spirit and scope of the present technology. In this regard, the present technology encompasses numerous additional features in addition to those specific features set forth in the paragraphs below. Moreover, the foregoing disclosure should be taken by way of illustration rather than by way of limitation as the present technology is defined by the paragraphs set forth below.

## Claims

1. A prosthetic femoral component, comprising:
an articular component (150, 450, 850) with a convex articular surface (154, 454, 854) and a concave curved inner surface (152, 452, 852) opposite the convex articular surface (154, 454, 854); and
a bone contacting component (100, 200, 300, 400, 600, 700, 800) at least partially insertable within the articular component (150, 450, 850), the bone contacting component (100, 200, 300, 400, 600, 700, 800) including an outer side and an inner side (102, 202, 302, 402, 602, 702a, 702b, 802) opposite the outer side, the outer side having a convex curved surface (104, 204, 304, 404, 604, 704a, 704b, 804) received on and complementary to the concave curved inner surface (152, 452, 852) of the articular component (150, 450, 850), and the inner side (102, 202, 302, 402, 602, 702a, 702b, 802) of the bone contacting component (100, 200, 300, 400, 600, 700, 800) being defined by a plurality of adjacent planar segments (112a-e, 212a-e, 312a-e, 412a-e, 612a-e, 712a-e, 812a-e).

2. The prosthetic femoral component of claim 1, wherein the articular component (150, 450, 850) includes a post (170a, 170b, 470a, 470b, 870a, 870b) extending from the concave curved inner surface (152, 452, 852), the post (170a, 170b, 470a, 470b, 870a, 870b) configured to extend through an aperture (116a, 116b, 216a, 216b, 316a, 316b, 416a, 416b, 616a, 616b, 716a, 716b, 816a, 816b) of the bone contacting component (100, 200, 300, 400, 600, 700, 800).

3. The prosthetic femoral component of claim 2, wherein the post (170a, 170b, 470a, 470b, 870a, 870b) is integrally formed with the articular component (150, 450, 850).

4. The prosthetic femoral component of claim 2 or claim 3, wherein the aperture (116a, 116b, 216a, 216b, 316a, 316b, 416a, 416b, 616a, 616b, 716a, 716b, 816a, 816b) of the bone contacting component (100, 200, 300, 400, 600, 700, 800) is an elongate slot sized such that while the post (170a, 170b, 470a, 470b, 870a, 870b) is received through the elongate slot, the bone contacting component (100, 200, 300, 400, 600, 700, 800) may translate relative to the post (170a, 170b, 470a, 470b, 870a, 870b).

5. The prosthetic femoral component of any one of claims 1-4, wherein the plurality of adjacent planar segments (112a-e, 212a-e, 312a-e, 412a-e, 612a-e, 712a-e, 812a-e) that define the inner side (102, 202, 302, 402, 602, 702a, 702b, 802) of the bone contacting component (100, 200, 300, 400, 600, 700, 800) include five adjacent planar segments (112a-e, 212a-e, 312a-e, 412a-e, 612a-e, 712a-e, 812a-e).

6. The prosthetic femoral component of any one of claims 1-5, wherein at least one planar segment of the plurality of adjacent planar segments (112a-e, 312a-e, 612a-e) includes a recessed surface region (114a-e, 314a-e, 614).

7. The prosthetic femoral component of anyone of claims 1-6, wherein the anterior component (450) includes an anterior flange (456a) extending higher than the anterior portion (406) of the bone contacting component (400), the anterior flange (456a) configured to flex to engage a bone.

8. The prosthetic femoral component of any one of claims 1-7, wherein the bone contacting component (300) includes a plurality of pores (318) extending at least partially through the bone contacting component (300) from the inner side (302) to the outer side (304), the plurality of pores (318) defining a grid pattern over a portion of at least one of the respective inner and outer sides (302, 304) of the bone contacting component (300).

9. The prosthetic femoral component of any one of claims 1-8, wherein the bone contacting component (700) includes an anterior component (700b) and a posterior component (700a) removably attachable to the anterior component (700b), the posterior component (700a) includes two condylar portions (708a, 708b) connected by a bridge (707).

10. The prosthetic femoral component of claim 9, wherein the anterior component (700b) includes one of a protrusion (705) and a recess (709) and the bridge (707) of the posterior component (700a) includes the other of the protrusion (705) and the recess, the protrusion (705) configured to be received in the recess (709) to connect the anterior component (700b) with the posterior component (700a).

11. The prosthetic femoral component of claim 10, wherein the posterior component (700a) is configured to hinge relative to the anterior component (700b) when the protrusion (705) is received in the recess (709).

12. The prosthetic femoral component of any one of claims 9-11, wherein when the posterior component (700a) is connected to the anterior component (700b), the posterior component (700a) is lockable in a plurality of angular orientations relative to the anterior component (700b).

13. A femoral implant kit, comprising:
a first bone contacting component (100, 200, 300, 400, 600, 700, 800) including a first inner surface (102, 202, 302, 402, 602, 702a, 702b, 802) having a first plurality of adjacent planar segments (112a-e, 212a-e, 312a-e, 412a-e, 612a-e, 712a-e, 812a-e) and a first outer surface (104, 204, 304, 404, 604, 704a, 704b, 804) opposite the first inner surface (102, 202, 302, 402, 602, 702a, 702b, 802);
a second bone contacting component (100, 200, 300, 400, 600, 700, 800) including a second inner surface (102, 202, 302, 402, 602, 702a, 702b, 802) having a second plurality of adjacent planar segments (112a-e, 212a-e, 312a-e, 412a-e, 612a-e, 712a-e, 812a-e) and a second outer surface (104, 204, 304, 404, 604, 704a, 704b, 804) opposite the second inner surface (102, 202, 302, 402, 602, 702a, 702b, 802), the second inner surface (102, 202, 302, 402, 602, 702a, 702b, 802) being different from the first inner surface (102, 202, 302, 402, 602, 702a, 702b, 802); and
an articular component (150, 450, 850) including an inner curved surface (152, 452, 852) with a post (170a, 170b, 470a, 470b, 870a, 870b) thereon,
wherein the first outer surface (104, 204, 304, 404, 604, 704a, 704b, 804) and the second outer surface (104, 204, 304, 404, 604, 704a, 704b, 804) are complementary to the inner curved surface (152, 452, 852) of the articular component (150, 450, 850) and the first and second bone contacting components (100, 200, 300, 400, 600, 700, 800) are configured to be received in the articular component (150, 450, 850).

14. The kit of claim 13, wherein the first and second bone contacting components (100, 200, 300, 400, 600, 700, 800) include respective first and second internal openings (116a, 116b, 216a, 216b, 316a, 316b, 416a, 416b, 616a, 616b, 716a, 716b, 816a, 816b) therethrough such that both the first and second bone contacting components (100, 200, 300, 400, 600, 700, 800) are independently receivable on the inner curved surface (152, 452, 852) of the articular component (150, 450, 850) by passing the internal opening (116a, 116b, 216a, 216b, 316a, 316b, 416a, 416b, 616a, 616b, 716a, 716b, 816a, 816b) over the post (170a, 170b, 470a, 470b, 870a, 870b), the internal opening (116a, 116b, 216a, 216b, 316a, 316b, 416a, 416b, 616a, 616b, 716a, 716b, 816a, 816b) having a shape such that the first or second bone contacting component (100, 200, 300, 400, 600, 700, 800) is rotatable relative to the articular component (150, 450, 850) while the outer surface (104, 204, 304, 404, 604, 704a, 704b, 804) of the first bone contacting component (100, 200, 300, 400, 600, 700, 800) is adjacent to the inner surface (152, 452, 852) of the articular component (150, 450, 850).

15. The kit of claim 13, wherein the first bone contacting component (700) further comprises a posterior component (700a) and an anterior component (700b) such that in an assembled state, the posterior component (700a) and the anterior component (700b) are connected to define the first bone contacting component (700).
